# EUROPÄISCHE PATENTSCHRIFT

(11) **EP 1 413 258 B1**
(45) Veröffentlichungstag und Bekanntmachung des Hinweises auf die Patenterteilung: **23.03.2005**
(21) Anmeldenummer: 02023790.5
(22) Anmeldetag: 25.10.2002
(51) Int. Cl.: A61B 19/00

(54) **Vorrichtung und Verfahren zum Kalibrieren eines Elements**
Device and method for calibrating an element
Appareil et procédé pour calibrer un élement

(43) Veröffentlichungstag der Anmeldung: 28.04.2004
(73) Patentinhaber: BrainLAB AG, 85551 Kirchheim/Heimstetten (DE)
(72) Erfinder: Blau, Arno, 86356 Neusäss (DE); Seifferth, Falko, 85604 Zorneding (DE)
(74) Vertreter: Schwabe - Sandmair - Marx

(56) Entgegenhaltungen:
- EP-A- 0 904 735
- WO-A-01/67979
- WO-A-02/061371
- US-A1- 2002 010 384

## Beschreibung

Die Erfindung bezieht sich auf ein Verfahren zum Kalibrieren eines zum Beispiel gebogenen oder nicht rotationssymmetrischen Elements oder Instruments, wie zum Beispiel eines Implantats, einer Führungshülse und/oder eines Halteelements, wobei das Element oder Instrument mit mindestens einem Navigationselement verbunden ist und in mindestens zwei und bevorzugt mehreren unterschiedlichen Positionen an eine Kalibriervorrichtung angelegt wird. DE-196 39 615 offenbart ein Verfahren, wonach die Position der Spitze eines Instruments durch Aufsetzen und bewegen bestimmt wird. Die Kalibriervorrichtung weist eine oder mehrere Oberflächen auf, deren Verlauf oder räumliche Position bekannt ist. Die Kalibriervorrichtung kann beispielsweise eine Ebene sein, deren räumliche Position bekannt ist. Die räumliche Position des Elements oder Instruments kann zunächst an mindestens einem Punkt ermittelt werden, welcher mit dem mindestens einen Navigationselement verbunden ist. Wird das Element oder Instrument auf die Ebene gelegt und so über die Ebene bewegt oder gedreht, dass das Element oder Instrument immer mit mindestens einem Punkt auf der Ebene aufliegt, so kann eine Vielzahl von Messwerten ermittelt werden, aus welchen sich bei einer hinreichend großen Anzahl unterschiedlicher Anlagepositionen der Verlauf des gebogenen Elements oder Instruments bzw. dessen Geometrie ermitteln lässt. Mathematisch betrachtet kann vor dem Kalibrieren der Verlauf oder die Geometrie des Elements oder Instruments als der gesamte dreidimensionale Raum angesehen werden. Jeder erfasste Messwert, welcher eine bestimmten Anlageposition des Elements oder Instruments an der bekannten Ebene darstellt, kann als eine Einschränkung der Möglichkeiten des dreidimensionalen Verlaufs des zu kalibrierenden Elements oder Instruments gesehen werden. Prinzipiell kann ein erfasster Messwert in einer bestimmten Anlageposition des Instruments oder Elements relativ zu einer Anlageebene so aufgefasst werden, dass die Anlageebene eine Tangentialebene an dem zu kalibrierenden Instrument oder Element ist und diese Tangentialebene ist die Grenzfläche eines Halbraumes, welcher für den räumlichen Verlauf des Instruments oder Elements nicht in Betracht kommt und somit aus dem ursprünglich zur Verfügung stehenden dreidimensionalen Raum weggeschnitten werden kann. Werden mehrere Anlagepositionen des Instruments oder Elements an der Ebene erfasst, so können mehrere Teilräume aus dem ursprünglich zur Verfügung stehenden dreidimensionalen Raum herausgeschnitten werden, so dass nach einer hinreichend großen Anzahl von Messwerten oder Kontaktpositionen der räumliche Verlauf des gebogenen Elements oder Instruments ermittelt werden kann.

Unter dem Begriff "Kalibrieren" soll das Bestimmen der Geometrie oder allgemein des räumlichen Verlaufs eines Körpers, wie zum Beispiel eines Instruments oder Elements verstanden werden.

Vorteilhaft kann in Verbindung mit dem oben beschriebenen Verfahren eine Plausibilitätskontrolle durchgeführt werden, wenn zum Beispiel bestimmte Annahmen bezüglich der Geometrie oder des räumlichen Verlaufs des zu kalibrierenden Instruments oder Elements während des Kalibrier-Verfahrens berücksichtigt werden können. Diese Annahmen oder Informationen können entweder fest eingestellt oder von einem Benutzer vorgegeben werden. Beispielsweise kann angenommen werden, dass das zu kalibrierende Element oder Instrument keinen Knick aufweist, einen im Wesentlichen konstanten bekannten oder noch zu bestimmenden Durchmesser hat, in nur eine Richtung gebogen ist, usw.

Bevorzugt kann die Spitze eines zu kalibrierenden Instruments oder Elements vor Durchführung des Kalibrier-Verfahrens auf bekannte Art kalibriert werden, d. h. zusätzlich zu dem Punkt des zu kalibrierenden Instruments oder Elements, welcher mit dem Navigationselement verbunden ist, ist die räumliche Position eines weiteren Punktes des zu kalibrierenden Instruments oder Elements bekannt, wodurch das Kalibrier-Verfahren vereinfacht werden kann. Das Kalibrieren der Spitze kann zum Beispiel dadurch erfolgen, dass die Spitze zu einer räumlichen Position gebracht wird, welche bekannt ist.

Vorteilhaft kann während des Kalibrier-Verfahrens die aus den bisher erfassten Daten oder Positionen ermittelte Form des zu kalibrierenden Elements angezeigt werden, so dass überprüft werden kann, ob prinzipiell die richtige Form des Instruments oder Elements erfasst wird, oder ob beim Kalibrieren ein Fehler aufgetreten ist.

Weiterhin bezieht sich die Erfindung auf eine Vorrichtung zum Kalibrieren eines beispielsweise gebogenen Elements oder Instruments, wobei an der Vorrichtung mindestens ein Navigationselement, wie zum Beispiel ein Referenzstern oder zwei, drei oder mehr Marker angebracht sind, so dass die räumliche Lage der Vorrichtung zum Kalibrieren erfasst werden kann.

Die Vorrichtung kann beispielsweise eine ebene Oberfläche aufweisen, an welche das zu kalibrierende Instrument oder Element angelegt werden kann. Ebenso kann die Vorrichtung zwei oder mehrere aneinander angrenzende Ebenen aufweisen, welche beispielsweise eine Kante oder eine V-Form bilden. Das zu kalibrierende Element oder Instrument kann dann in das durch die Ebenen gebildete V eingebracht werden, so dass es mit jeder der das V bildenden Ebenen mindestens einen Berührungspunkt hat, wodurch das Kalibrier-Verfahren vereinfacht wird.

Die Vorrichtung zum Kalibrieren eines Instruments oder Elements weist ein bewegbares Element auf, welches mit der Vorrichtung verbunden ist und relativ zur Vorrichtung bewegt, wie zum Beispiel geklappt oder geschwenkt werden kann. Das bewegbare Element ist dabei mit mindestens einem Marker oder Navigationselement verbunden. Bevorzugt ist das bewegbare Element so angeordnet, dass es mit mindestens einem weiteren Punkt des auf der Vorrichtung aufliegenden zu kalibrierenden Elements oder Instruments Kontakt haben kann, wodurch das Kalibrieren des Instruments weiter verbessert werden kann. Beispielsweise kann das bewegbare Element so angeordnet sein, dass es durch eine Feder oder durch eigenes Gewicht eine Druckkraft in Richtung der Kalibrier-Vorrichtung ausübt, wodurch ein zu kalibrierendes Element oder Instrument, welches an der Vorrichtung anliegt, an einer der Vorrichtung abgewandten Seite in Kontakt mit dem bewegbaren in Richtung der Vorrichtung drückenden Elements kommt. Dies ermöglicht das schnellere und genauere Kalibrieren eines Elements oder Instruments.

Die Position des zum Beispiel durch eine Feder vorgespannten bewegbaren Elements oder eines Federelements kann durch einen darauf angeordneten Marker ermittelt und zum Beispiel in senkrechter Richtung getrackt werden. Mathematisch betrachtet kann man annehmen, dass über dem Federelement, das durch die Kraft F auf das zu kalibrierende Element (Instrument oder Implantat) gedrückt wird, ein Quader mit unendlicher Ausdehnung nach oben, unendlicher Ausdehnung senkrecht zur V-Nut (zum Beispiel parallel zur Oberfläche der Kalibrier-Vorrichtung) und einer Ausdehnung parallel zur V-Nut mit der Breite (parallel zur V-Nut) des Federelements vorliegt, der von dem noch verbliebenen, als zum Instrument oder Implantat gehörend betrachteten Raum, abgezogen wird. D. h. der für die Geometrie das zu kalibrierenden Instruments zur Verfügung stehende Raum wird durch den so erzeugten Quader verringert.

Der Kalibrier-Vorgang kann beispielsweise zeitlich begrenzt sein, so dass nach einer Anfangszeit nur über einen vorgegebenen Zeitraum hinweg Daten gesammelt werden. Während des Kalibrierens sollte das zu kalibrierende Instrument oder Element immer an der Kalibrier-Vorrichtung anliegen, d. h. mindestens einen Kontaktpunkt haben und es sollte vorteilhaft mindestens um zum Beispiel 180° gedreht werden. Wurden während des Kalibrierens ausreichend viele Daten zum Spezifizieren der Geometrie des Instruments oder Elements gesammelt, welche einem Mindest-Winkelbereich oder Mindest-Drehbereich des Instruments oder Elements entsprechen, so kann die Geometrie oder der räumliche Verlauf des Instruments oder Elements bestimmt werden, wodurch dieses kalibriert ist.

Weist das zu kalibrierende Element oder Instrument eine einfache Geometrie auf, d. h. ist das Instrument beispielsweise ein gerader Stab, so ist das Verfahren zum Kalibrieren des Instruments relativ einfach durchzuführen, da der Stab beispielsweise nur auf eine ebene Kalibrier-Vorrichtung aufgelegt und um seine Längsachse gedreht werden muss, wodurch bereits der räumliche Verlauf des Stabes relativ zu dem mit diesem Stab verbundenen Navigationselement bekannt ist. Instrumente oder Elemente mit einer komplexeren Struktur müssen in mehreren Positionen anliegend an der Kalibrier-Vorrichtung bewegt werden, um deren Geometrie mit einer hinreichenden Genauigkeit beschreiben zu können.

Weiterhin bezieht sich die Beschreibung auf ein Computerprogramm, welches wenn es in einen Computer geladen ist oder auf einem Computer läuft, mindestens einen in dieser Anmeldung beschriebenen Verfahrensschritte ausführt sowie auf ein Programm-Speicher-Medium mit einem solchen Computerprogramm.

Die vorliegende Beschreibung bezieht sich des Weiteren auf eine Vorrichtung zum vorzugsweise minimal invasiven Einbringen eines Elements, wie zum Beispiel eines Stabes in einen Körper, wobei dieses Element vorteilhaft durch ein Navigationsverfahren an eine bestimmte Position gebracht und mit Pedikelschrauben insbesondere bei Eingriffen im Bereich der Wirbelsäule verbunden werden kann. Weiterhin bezieht sich die Erfindung auf ein Verfahren zum Kalibrieren eines Elements, zum Beispiel eines gebogenen Implantats oder Instruments insbesondere eines chirurgischen Instruments, welches bei Operationen im Bereich der Wirbelsäule verwendet werden kann.

Bei Eingriffen oder Operationen im Bereich der Wirbelsäule wird zum Anbringen von Pedikelschrauben an einzelnen Wirbeln und zum Befestigen von Stäben zwischen Pedikelschrauben benachbarter Wirbel der gesamte Bereich der zu operierenden Wirbelsäule offengelegt. In die offengelegten Wirbel werden zunächst die Pedikelschrauben eingebracht, wonach jeweils ein Stab zwischen zwei Pedikelschrauben benachbarter Wirbel gelegt und mit diesen Schrauben verbunden wird. Anschließend wird das geöffnete Gewebe wieder über den eingebrachten Pedikelschrauben und den mit diesen verbundenen Stäben geschlossen. Ein solcher Eingriff im Bereich der Wirbelsäule erfordert demzufolge das Öffnen des Gewebes in einem relativ großen Bereich.

Aus der US 6,371,957 B1 ist eine Pedikelschraube mit einer darauf angeordneten Vorrichtung zum Befestigen eines Stabes bekannt.

Die Öffnung eines relativ großen Gewebebereiches bringt jedoch Probleme mit sich, wie zum Beispiel eine erhöhte Infektionsgefahr oder Komplikationen beim Heilen des entsprechenden Bereiches. Des Weiteren können nach solchen Eingriffen Narben vorhanden sein, was aus ästhetischen Gründen unerwünscht ist.

Weiters wird eine Vorrichtung zum Einbringen eines zum Beispiel stabförmigen geraden oder gebogenen Elements in einen Körper vorgeschlagen, welche das minimal invasive Einbringen und/oder Positionieren eines solchen Stabes ermöglicht. Weiterhin sollen ein Verfahren und eine Vorrichtung vorgeschlagen werden, mit welchen ein zum Beispiel gebogenes Instrument kalibriert werden kann.

Gemäß einem ersten Aspekt weist eine Führungshülse, welche zum Beispiel zur Führung eines später beschriebenen Halteelements verwendet werden kann, eine Eingangsöffnung und eine Ausgangsöffnung auf, durch welche ein Halteelement geschoben werden kann. Zwischen der Eingangsöffnung und der Ausgangsöffnung liegt ein Führungsbereich, in welchem das Halteelement so geführt werden kann, dass das Halteelement durch die Führungshülse geschoben und zum Beispiel in diesem gedreht werden kann. Die Führungshülse kann zum Beispiel in einer einfachen Ausführungsform ein ringförmiges oder röhrenförmiges geradlinig verlaufendes oder gebogenes Element sein, durch welches das Halteelement geschoben und geführt werden kann. Bevorzugt ist die Führungshülse starr, so dass die Führungshülse nicht leicht verformt werden kann und somit ein sicheres und definiertes Führen des Halteelements gewährleistet ist. Die Führungshülse kann jedoch auch aus einem elastischen Material oder einer Kombination aus elastischen und starren Teilstücken bestehen, um die Form der Führungshülse verändern und an eine bestimmte Anwendung anpassen zu können. Dabei sollte ein für die Führungshülse verwendetes elastisches oder verformbares Material vorzugsweise eine gewisse Beständigkeit gegen Verformung durch äußere Kräfte aufweisen, um ein unbeabsichtigtes Verformen der Führungshülse und damit das Führen des Halteelements zu einer nicht gewünschten Position zu verhindern.

Bevorzugt ist die Führungshülse gebogen oder weist mindestens einen gebogenen oder gekrümmten Teilbereich auf.

Vorteilhaft ist an der Führungshülse ein Befestigungsbereich für ein Navigationselement, wie zum Beispiel ein Referenzstern oder eine andere Kombination einer oder mehrerer aktiver oder passiver Marker vorgesehen. Die Verwendung von Navigationselementen, wie zum Beispiel Markern zum Navigieren und Positionieren von Instrumenten ist im Stand der Technik bekannt und wird hier nicht näher beschrieben. Eine mit einem Navigationselement verbundene Führungshülse ermöglicht das Navigieren eines in der Führungshülse geführten Halteelements und/oder eines mit dem Halteelement verbundenen weiteren Elements, wie zum Beispiel eines Stabes, welcher beispielsweise zwischen zwei, drei oder mehreren Pedikelschrauben positioniert werden soll.

Vorteilhaft kann ein Navigationselement, wie zum Beispiel ein Referenzstern, auf die Führungshülse mit einem Aufschiebeelement aufgeschoben werden, so dass die Führungshülse relativ zum Navigationselement bewegbar ist. Die Führungshülse kann dann zum Beispiel durch ein in etwa ortsfest gehaltenes mit einem Navigationselement verbundenes Aufschiebeelement hindurchgeschoben werden, so dass im Fall einer bekannten konstanten Krümmung der Führungshülse ein durch die Führungshülse geführtes Element an einer vorgegebenen gewünschten Stelle positioniert werden kann.

Bevorzugt weist die Führungshülse an dem Ende, welches mit einem zu positionierenden Element, wie zum Beispiel einem Implantat oder Stab, in Anlage kommen soll, eine Geometrie und/oder eine Oberfläche auf, welche ein Verrutschen des zu positionierenden Elements erschweren oder verhindern. Beispielsweise kann ein Ende der Führungshülse nach außen oder nach innen konisch zulaufend ausgebildet sein, um mit einem entsprechend ausgebildeten Ende eines zu positionierenden Elements für einen Halt des Elements zu sorgen. Insbesondere ist es vorteilhaft eine Verdrehsicherung, wie zum Beispiel eine strukturierte Oberfläche oder vorstehende Elemente im Kontaktbereich zu einem Implantat vorzusehen, um dieses sicher halten und bewegen zu können.

Weiterhin ist ein Halteelement vorgesehen, welches zum Beispiel durch die oben beschriebene Führungshülse geführt und beispielsweise hindurchgeschoben und in dieser gedreht werden kann, wobei das Halteelement ein Verbindungselement aufweist, welches mit einem zu positionierenden Element, wie zum Beispiel einem Implantat oder Stab verbunden werden kann oder welches das zu positionierende Element zum Beispiel durch Kraftschluss oder Klemmen halten kann.

Gemäß einer vorteilhaften Ausführungsform weist das Halteelement mindestens einen starren und/oder einen flexiblen oder biegbaren Bereich auf und ist zum Beispiel ein Stab, welcher starr oder flexibel sein kann und sich bevorzugt zum Führen durch die Führungshülse eignet. Vorteilhaft hat ein solcher Stab einen Außendurchmesser, welcher etwas kleiner ist als der Innendurchmesser der Führungshülse, um ein einfaches Hindurchschieben des Stabes durch die Führungshülse sicherzustellen.

Das zum Verbinden des Halteelements mit einem zu positionierenden Element vorgesehene Verbindungselement kann beispielsweise ein Außengewinde oder ein Innengewinde sein, welches in ein korrespondierendes Innengewinde oder ein Außengewinde des zu führenden Elements oder Implantats eingreifen kann, um somit eine Verbindung zu diesem Element herzustellen. In diesem Fall ist es vorteilhaft das Halteelement und die Führungshülse so auszugestalten, dass das Halteelement in der Führungshülse nicht nur verschoben, sondern auch gedreht werden kann.

Vorteilhaft weist das Halteelement an dem dem zum Halten des zu positionierenden Elements gegenüberliegenden Ende einen Griff und/oder ein Gewinde auf, auf welches eine Mutter oder ein anderes geeignetes Element aufgeschraubt werden kann, um das in die Führungshülse eingebrachte Halteelement relativ zur Führungshülse durch ein Einspannen der Führungshülse zwischen zwei Bereichen oder den Enden des Halteelements fixieren zu können. Alternativ oder ergänzend können auch andere Haltemechanismen, wie zum Beispiel Klammern am Halteelement und/oder an der Führungshülse vorgesehen sein, um ein Fixieren des Halteelements relativ zur Führungshülse zu ermöglichen.

Nach einem weiteren Aspekt weist ein zu positionierendes Element, wie zum Beispiel ein Implantat oder ein Stab, welcher zwischen zwei Pedikelschrauben positioniert werden soll, mindestens ein Verbindungselement auf, welches mit einem korrespondierenden Verbindungselement des Halteelements zusammenwirken kann, um das zu positionierende Element mit dem Halteelement zu verbinden. Das Verbindungselement kann beispielsweise ein Innengewinde oder ein Außengewinde sein.

Bevorzugt ist das zu positionierende Element an dem Ende konisch zulaufend oder spitz ausgebildet, welches dem Ende des Elements gegenüberliegt, bei welchem das Verbindungselement vorgesehen ist, so dass die Spitze oder ein konisch zulaufender Bereich das Einbringen des zu positionierenden Elements in einen Körper vereinfacht.

Vorteilhaft ist das zu positionierende Element im Bereich des Verbindungselements so ausgestaltet, dass es möglichst verschiebesicher von einem Halteelement und/oder einer Führungshülse gehalten werden kann. Dies kann zum Beispiel ein nach außen oder nach innen konisch zulaufender Bereich des zu positionierenden Elements sein.

Gemäß einem weiteren Aspekt bezieht sich die Beschreibung auf ein System mit einer wie oben beschriebenen Führungshülse und einem wie oben beschriebenen Halteelement. Vorteilhaft weist das System weiterhin ein wie oben beschriebenes zu positionierendes Element auf, wobei bevorzugt das Halteelement durch die Führungshülse hindurchgeschoben werden kann und mit dem zu positionierenden Element verbunden werden kann.

Ein solches System ermöglicht das minimal invasive navigierte genaue Positionieren eines Elements oder Implantats, welches durch eine relativ kleine Öffnung durch Gewebe hindurch in einen Körper eingebracht und in diesem an einer gewünschten Stelle unter Verwendung des mit der Führungshülse und/oder dem Halteelement verbundenen Navigationselements positioniert werden kann, wobei sowohl das Halteelement als auch die Führungshülse nach dem Lösen der Verbindung zwischen Halteelement und/oder Führungshülse einerseits und dem zu navigierenden Implantat oder Element, wie zum Beispiel einem Stab andererseits wieder aus dem Körper herausgenommen werden können.

Die Erfindung wird nachfolgend anhand bevorzugter Ausführungsbeispiele beschrieben. Es zeigen:
- Figur 1: eine erfindungsgemäße Führungshülse mit Referenzstern;
- Figur 1A: eine vergrößerte Detailansicht des vorderen Endes der in Figur 1 gezeigten Führungshülse;
- Figur 2: eine Ausführungsform eines erfindungsgemäßen Halteelements;
- Figur 3: ein aus den in den Figuren 1 und 2 gezeigten Elementen zusammengesetztes erfindungsgemäßes System mit einem Implantat;
- Figur 3A: eine vergrößerte Detailansicht des in Figur 3 gezeigten Ausschnitts A;
- Figur 4: eine alternative Ausführungsform eines erfindungsgemäßen Systems; und
- Figur 5: eine erfindungsgemäße Kalibrier-Vorrichtung.

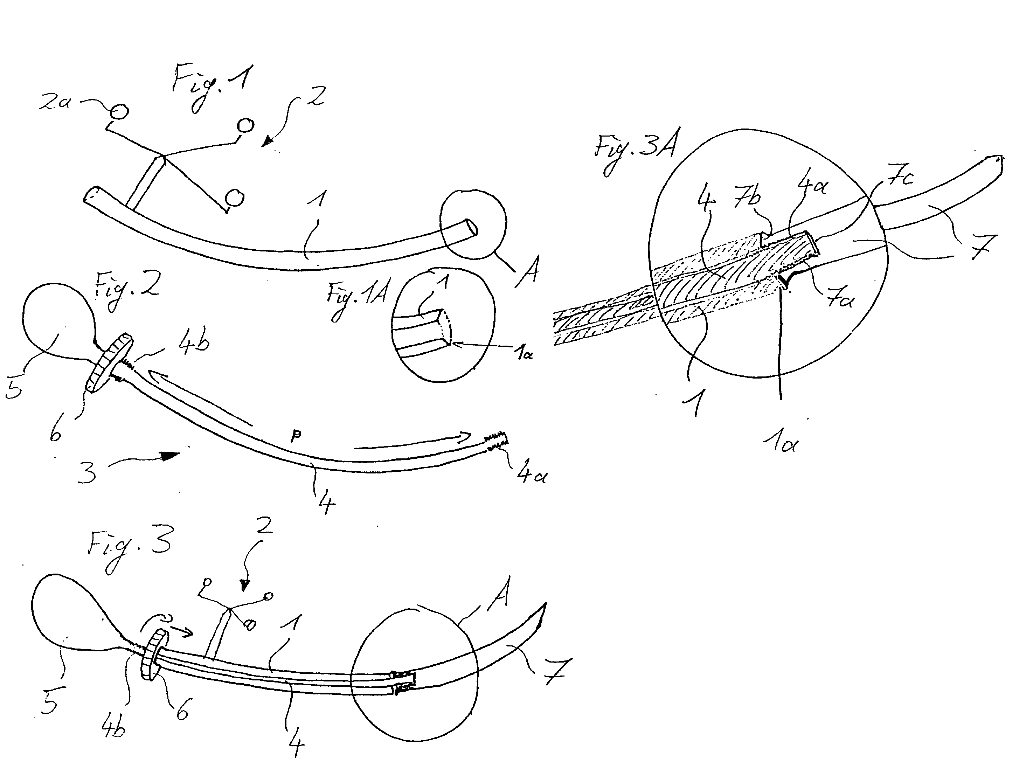

Figur 1 zeigt eine röhrenförmige Führungshülse 1, welche mit einem Referenzstern 2, der drei passive Marker 2a aufweist, verbunden ist. Die Führungshülse 1 ist an dem in Figur 1 links gezeigten hinteren und rechts gezeigten vorderen Ende offen. Figur 1A zeigt den in Figur 1 mit A bezeichneten Teil der Führungshülse 1 in vergrößerter Darstellung im Querschnitt. Die Führungshülse 1 weist, wie in Figur 1A gezeigt, einen in etwa konisch verlaufenden Randbereich 1a auf, auf welchen ein Implantat 7 aufgesetzt werden kann, wie in Figur 3 gezeigt.

Figur 2 zeigt ein Halteelement 3, welches über den durch die Pfeile P gezeigten Teilbereich 4 elastisch oder flexibel ist. Der Teilbereich 4 kann beispielsweise ein flexibles Metall- oder Kunststoffelement sein, welches im vorderen in Figur 2 rechts gezeigten Ende ein Außengewinde 4a aufweist. Das in Figur 2 links gezeigte hintere Ende des flexiblen Bereichs 4 weist ebenfalls ein Außengewinde 4b auf, auf welches eine Mutter 6 aufgeschraubt ist. An das Gewinde 4b schließt sich ein Griff 5 an, welcher zum Halten des Halteelements 3 dient.

Figur 3 zeigt das in die Führungshülse 1 von Figur 1 eingesetzte in Figur 2 gezeigte Halteelement 3, wobei der flexible Bereich 4 durch die Führungshülse 1 hindurchgeführt wurde. Am vorderen Ende ist das Implantat 7 auf das vordere Außengewinde 4a des Halteelements 3 aufgeschraubt.

Figur 3A zeigt die vergrößerte Querschnittsansicht des mit A bezeichneten Teils aus Figur 3. Das Implantat 7 ist an seinem rechts gezeigten vorderen Ende zugespitzt und weist an seinem hinteren Ende ein Innengewinde 7a auf, mit welchem das Implantat 7 auf das Außengewinde 4a des flexiblen Teils 4 des Halteelements 3 aufgeschraubt ist. Weiterhin ist das hintere Ende des Implantats 7 im Bereich 7b konisch zulaufend und liegt somit verschiebungssicher an dem korrespondierenden konischen Bereich 1a der Führungshülse 1 an.

Zum Zusammensetzen des in Figur 3 gezeigten Systems sollte, wie in Figur 2 gezeigt, die Mutter 6 in Richtung des Griffes 5 an den äußeren Bereich des Außengewindes 4b geschraubt werden. Das Halteelement 3 kann in die Führungshülse 1 eingebracht werden. In dieser Position kann das Implantat 7 auf das vordere Außengewinde 4a vorzugsweise bis zum Anschlag des vorderen Bereichs des flexiblen Elements 4 an der inneren Begrenzung 7c des Implantats 7 aufgeschraubt werden. Anschließend kann die Schraube 6 in Richtung des Implantats 7 so weit geschraubt werden, bis die Führungshülse 1 sicher zwischen der Schraube 6 und dem mit dem Halteelement 3 verbundenen Implantat 7 verspannt ist, so dass die einzelnen Komponenten des in Figur 3 gezeigten Systems im Wesentlichen nicht mehr relativ zueinander verrutschen können.

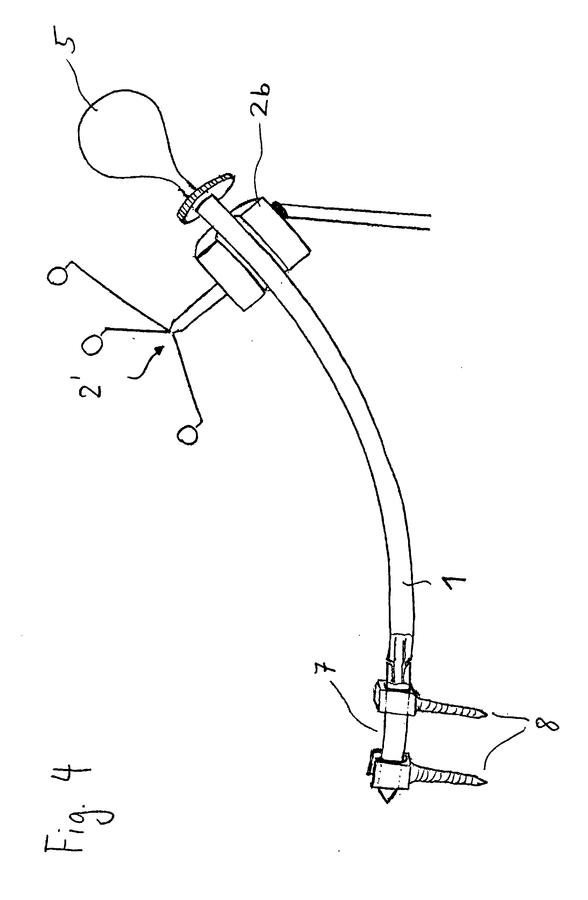

Das so zusammengesetzte System kann nun entweder durch einen fest mit der Führungshülse 1 verbundenen Referenzstern 2 oder durch einen über ein verschiebbares Element 2b angebrachten Referenzstern 2' - wie in Figur 4 gezeigt - navigiert werden. Dabei kann beispielsweise das Verschiebungselement 2b so gehalten werden, dass die Führungshülse 1 so durch das Verschiebungselement 2b hindurch bewegt werden kann, dass das mit der Führungshülse 1 über das Halteelement 3 verbundene Implantat 7 in eine gewünschte Position zwischen zwei Pedikelschrauben 8 gebracht wird, wie ebenfalls in Figur 4 gezeigt.

Ist das mit der Führungshülse 1 und dem Halteelement 3 verbundene Implantat 7 in eine gewünschte Position gebracht worden, so kann das Implantat 7 in dieser Position an den Pedikelschrauben 8 befestigt werden. Anschließend kann durch eine Drehbewegung des Halteelements 3 die Schraubverbindung zwischen dem Halteelement 3 und dem Implantat 7 gelöst werden, d. h. das Außengewinde 4a auf der Vorderseite des Halteelements 3 wird aus dem Innengewinde 7a des Implantats 7 herausgeschraubt. Vorteilhaft kann der Kontaktbereich zwischen Führungshülse 1 und Implantat 7 Elemente zur Verdrehsicherung aufweisen, wie zum Beispiel vorstehende Elemente, welche in kleine Nuten im hinteren Anlagebereich des Implantats 7 eingreifen, so dass eine Zugkraft am Halteelement 3 das Implantat 7 fest gegen die Führungshülse 1 drückt und somit das Außengewinde 4a des Halteelements 3 sicher aus dem Implantat 7 herausgeschraubt werden kann, ohne dabei die Position des Implantats 7 zu verändern.

Ist die Verbindung zwischen Halteelement 3 und Implantat 7 gelöst, so kann das Halteelement 3 zusammen mit der Führungshülse 1 aus dem Körper herausgenommen werden, wodurch das Implantat 7 durch ein minimal invasives Verfahren zu einer vorgegebenen Position navigiert worden ist.

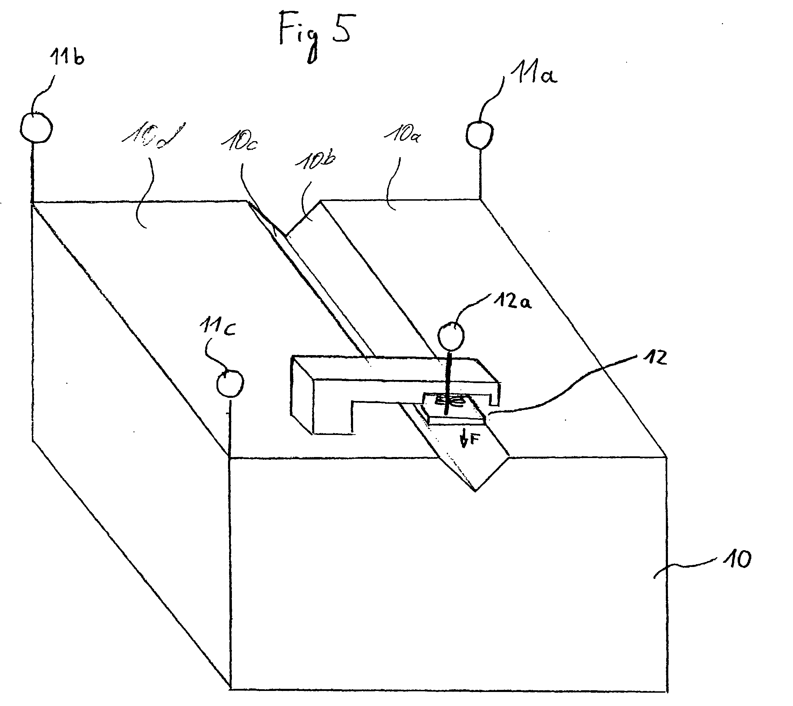

Figur 5 zeigt eine Kalibrier-Vorrichtung 10 mit drei daran angebrachten reflektierendenMarkern 11a, 11b und 11c. Die Oberseite der Kalibrier-Vorrichtung 10 besteht aus zwei in einer Ebene liegenden Flächen 10a und 10d, zwischen welchen zwei ein V bildende Ebenen 10b und 10c liegen. Auf der Oberseite 10d ist ein federndes Element 12 angebracht, welches in der durch den Pfeil F angezeigten Richtung auf die Oberseite des zu kalibrierenden Elements drückt, welches auf der Kalibrier-Vorrichtung 10 aufliegt.

Soll das in Figur 3 gezeigte System kalibriert werden, d. h. die Geometrie des Systems ermittelt werden, so kann dieses in die durch die Oberflächen 10b und 10c gebildete Rille oder V-Form gelegt und so bewegt werden, dass das in Figur 3 gezeigte System mindestens an einem Punkt der Oberflächen 10b und 10c anliegt. Werden mehrere Drehpositionen erfasst, so kann aufgrund der durch die Marker 11a, 11b und 11c bekannten Position der Kalibrier-Vorrichtung 10 in Verbindung mit der durch den Referenzstern 2 bekannten Position mindestens eines Punktes des in Figur 3 gezeigten Systems dieses System kalibriert werden. Das federnde oder elastische Element 12 weist einen reflektierenden Marker 12a auf und drückt aufgrund der Vorspannung durch Federkraft auf die Oberseite des in die V-Form eingebrachten zu kalibrierenden Instruments, wodurch ein weiterer Anlagepunkt entsteht, der über die Position des Markers 12a erfasst werden kann. Somit kann das in Figur 3 gezeigte System oder allgemein jedes Instrument oder Element kalibriert werden.

Zum Einbringen des Implantats 7 kann vorteilhaft eine prä-operative und eine intra-operative Planung durchgeführt werden.

Bei der prä-operativen Planung wird durch ein bildgebendes Verfahren, wie zum Beispiel MRI, die Position verschiedener Rückenwirbel erfasst. Die einzelnen Rückenwirbel werden durch ein bekanntes Verfahren segmentiert, d. h. es wird ermittelt wie die räumliche Begrenzung eines jeden Wirbels ist. Ein Chirurg kann basierend auf der erfassten (pathologischen) Position eine Neuanordnung der Rückenwirbel vornehmen, bis eine optimale biomechanische Position der Rückenwirbel zueinander erreicht ist. Dies kann zum Beispiel unter Verwendung von bekannten Simulationsprogrammen durchgeführt werden. Anschließend wird ermittelt an welchen Positionen vorteilhaft Pedikelschrauben mit einer bestimmten Abmessung angeordnet werden sollen. Basierend darauf kann ermittelt werden, welche Form ein zwischen die Pedikelschrauben 8 einzubringendes Implantat 7 haben muss. Die so ermittelte Form des Implantats kann dann zum Beispiel ausgedruckt werden, so dass das Implantat 7 zum Beispiel unter Verwendung des Ausdrucks als Vorlage geformt werden kann. Alternativ ist es auch möglich aus einer Anzahl fest vorgegebener Implantate das für den vorliegenden Fall geeignete auszuwählen.

Bei der intra-operativen Planung können die zu behandelnden Wirbel im CT Datensatz segmentiert werden, wobei dieser Schritt auch bereits prä-operativ durchgeführt werden kann. Jeder Wirbel wird einzeln registriert und mit einer Referenziervorrichtung wie zum Beispiel einem Marker oder einer Markeranordnung versehen, um ein fortlaufendes Tracken aller registrierten Wirbel zu ermöglichen. Wenn nun die Wirbelsäule zum Beispiel durch Verschieben einzelner Wirbel neu geformt wird, kann die neue Form (Relativposition der Wirbel untereinander) am Bildschirm dargestellt werden und mit einer zum Beispiel prä- oder auch intra-operativ geplanten Form abgeglichen werden. Mit dieser nun bekannten aktuellen Form und den bereits geplanten Pedikelschrauben kann nun die Form eines einzusetzenden Implantats, wie zum Beispiel eines Verbindungsstabes berechnet werden.

Anschließend kann das so ausgewählte oder geformte Implantat 7 mit der Führungshülse 1 und dem Halteelement 3 verbunden werden, wie in den Figuren 1 bis 3 gezeigt.

Eine Kalibrierung des Systems bestehend aus Führungshülse 1, Halteelement 3 und Implantat 7 kann unter Verwendung der in Figur 5 gezeigten Kalibrier-Vorrichtung 10 durchgeführt werden.

Anschließend kann das Implantat minimal invasiv zu der durch die prä-operative Planung ermittelten Position navigiert werden und in dieser Position nach einer Befestigung an Pedikelschrauben gehalten werden, während gleichzeitig das Halteelement 3 und die Führungshülse 1 von dem Implantat 7 gelöst werden, so dass das Implantat 7 minimal invasiv zu einer gewünschten Position navigiert werden kann.

## Patentansprüche

1. Verfahren zum Bestimmen des räumlichen Verlaufs eines nicht rotationssymmetrischen Elements (1, 3, 7), wobei das nicht rotations-symmetrische Element (1, 3, 7) mit einem Navigationselement (2, 2') verbunden ist, in mindestens zwei unterschiedlichen Positionen an eine Kalibrier-Vorrichtung (10) angelegt und in Anlage an dieser Kalibriervorrichtung (10) jeweils so bewegt wird, dass das Element (1, 3, 7) mit mindestens einem Punkt an der Kalibrier-Vorrichtung (10) aufliegt.

2. Verfahren nach Anspruch 1, wobei die Kalibrier-Vorrichtung eine Ebene oder ein mit Markern (11) verbundenes Instrument (10) ist.

3. Verfahren nach einem der vorhergehenden Ansprüche, wobei eine Plausibilitätsüberprüfung durchgeführt wird.

4. Verfahren nach einem der vorhergehenden Ansprüche, wobei eine Spitze des zu kalibrierenden Elements kalibriert wird.

5. Verfahren nach einem der vorhergehenden Ansprüche, wobei ein Durchmesser des zu kalibrierende Elements eingegeben und/oder berechnet wird.

6. Verfahren nach einem der vorhergehenden Ansprüche, wobei das Ergebnis des Kalibrier-Verfahrens optisch während oder nach dem Kalibrieren angezeigt wird.

7. Vorrichtung zur Bestimmung des räumlichen Verlaufs eines nicht rotationssymmetrischen Elements (1, 3, 7), zum Durchführen des Verfahrens nach einem der Ansprüche 1 bis 6, wobei die Vorrichtung (10) mit mindestens einem Navigationselement (11a, 11b, 11c) verbunden ist und ein Element (12) aufweist, welches mit einer Kraft in Richtung der Oberfläche der Vorrichtung (10) drückt und welches mit mindestens einem Navigationselement (12a) verbunden ist.

8. Vorrichtung nach dem vorhergehenden Anspruch, wobei die Vorrichtung eine durch mindestens zwei Ebenen (10b, 10c) gebildete Kante aufweiset.

## Claims

1. A method for determining the spatial trajectory of a rotationally asymmetrical element (1, 3, 7), wherein said rotationally asymmetrical element (1, 3, 7) is connected to a navigation element (2, 2'), in contact with a calibrating device (10) in at least two different positions, and moved - when respectively in contact with said calibrating device - such that the element (1, 3, 7) lies on the calibrating device (10) via at least one point.

2. The method as set forth in claim 1, wherein the calibrating device is a plane or an instrument (10) connected to markers (11).

3. The method as set forth in any one of the preceding claims, wherein a plausibility check is performed.

4. The method as set forth in any one of the preceding claims, wherein a tip of the element to be calibrated is calibrated.

5. The method as set forth in any one of the preceding claims, wherein a diameter of the element to be calibrated is inputted and/or calculated.

6. The method as set forth in any one of the preceding claims, wherein the result of said calibrating method is optically displayed during or after calibration.

7. A device for determining the spatial trajectory of a rotationally asymmetrical element (1, 3, 7), for performing the method as set forth in any one of claims 1 to 6, wherein said device (10) is connected to at least one navigation element (11a, 11b, 11c) and comprises an element (12) which presses with a force in the direction of the surface of the device (10) and which is connected to at least one navigation element (12a).

8. The device as set forth in the preceding claim, wherein the device comprises an edge formed by at least two planes (10b, 10c).

## Revendications

1. Procédé pour déterminer l'allure spatiale d'un élément ne présentant pas la symétrie de révolution (1, 3, 7), dans lequel l'élément ne présentant pas la symétrie de révolution (1, 3, 7) est relié à un élément de navigation (2, 2'), est appliqué dans au moins deux positions différentes sur un dispositif de calibrage (10) et est déplacé chaque fois en appui sur ce dispositif de calibrage (10) de telle façon que l'élément (1, 3, 7) s'appuie par au moins un point sur le dispositif de calibrage (10).

2. Procédé selon la revendication 1, dans lequel le dispositif de calibrage est un plan ou un instrument (10) relié à des repères de position (11).

3. Procédé selon l'une quelconque des revendications précédentes, dans lequel on effectue un contrôle de plausibilité.

4. Procédé selon l'une quelconque des revendications précédentes, dans lequel on calibre une pointe de l'élément à calibrer.

5. Procédé selon l'une quelconque des revendications précédentes, dans lequel on introduit et/ou on calcule un diamètre de l'élément à calibrer.

6. Procédé selon l'une quelconque des revendications précédentes, dans lequel on affiche optiquement le résultat de l'opération de calibrage pendant ou après le calibrage.

7. Dispositif pour la détermination de l'allure spatiale d'un élément ne présentant pas la symétrie de révolution (1, 3, 7), pour la mise en oeuvre du procédé selon l'une quelconque des revendications 1 à 6, dans lequel le dispositif (10) est relié à au moins un élément de navigation (11a, 11b, 11c) et présente un élément (12), qui presse avec une force en direction de la surface du dispositif (10) et qui est relié à au moins un élément de navigation (12a).

8. Dispositif selon la revendication précédente, dans lequel le dispositif présente une arête formée par au moins deux plans (10b, 10c).
